# EUROPEAN PATENT SPECIFICATION

(11) **EP 2 143 383 B1**
(45) Date of publication and mention of the grant of the patent: **18.04.2012**
(21) Application number: 09163660.5
(22) Date of filing: 24.06.2009
(51) Int. Cl.: A61B 8/08

(54) **Image Depth Setting in an Ultrasound System**
Bildtiefeneinstellung in einem Ultraschallsystem
Régulation de profondeur d'image dans un système à ultrasons

(30) Priority: 10.07.2008 KR 20080066780
(43) Date of publication of application: 13.01.2010
(73) Proprietor: Samsung Medison Co., Ltd., Kangwon-do 250-875 (KR)
(72) Inventor: Yang, Eun Ho, 135-851, Seoul (KR); Park, Sung In, 135-851, Seoul (KR); Song, Jung Sik, 135-851, Seoul (KR); Lee, Su Myeong, 135-851, Seoul (KR); Shin, Soo Hwan, 135-851, Seoul (KR)
(74) Representative: Schmid, Wolfgang

(56) References cited:
- WO-A-2004/056271
- US-A- 5 897 498
- US-A1- 2004 260 178
- US-A1- 2005 240 103
- US-B1- 6 464 636

## Description

### TECHNICAL FIELD

The present disclosure generally relates to an ultrasound system, and more particularly to image depth setting in an ultrasound system.

### BACKGROUND

Recently, an ultrasound system has been extensively used in the medical field due to its non-invasive and non-destructive nature. Modem high-performance ultrasound systems and techniques are commonly used to produce two-dimensional and three-dimensional ultrasound images of internal features of a target object.

Generally, the ultrasound system transmits/receives ultrasound signals to/from the target object, thereby outputting electrical receive signals. Also, the ultrasound system forms an ultrasound image of the target object by using the receive signals depending on an image depth, which represents the range of an ultrasound image of the target object to be formed in a depth direction.

US 2005/240103 A1 describes an ultrasound imaging system provided with an interface for receiving user input, and a controller coupled to the interface, the controller being adapted and configured to adjust parameters for a catheter-based ultrasound probe in response to received user input. A user request for a desired imaging depth is received, an imaging frequency corresponding to the desired imaging depth is automatically determined and an imaging frequency of the system is adjusted to the determined imaging frequency.

### SUMMARY

Embodiments for image depth setting are disclosed herein. According to the invention, there is provided an ultrasound system according to claim 1 and a method of setting an image depth in an ultrasound system according to claim 6.

The Summary is provided to introduce a selection of concepts in a simplified form that are further described below in the Detailed Description. This Summary is not intended to identify key or essential features of the claimed subject matter, nor is it intended to be used in determining the scope of the claimed subject matter.

### BRIEF DESCRIPTION OF THE DRAWINGS

FIG 1 is a block diagram showing an illustrative embodiment of an ultrasound system.
FIG 2 is a flowchart showing a procedure of setting an image depth.

### DETAILED DESCRIPTION

A detailed description may be provided with reference to the accompanying drawing. One of ordinary skill in the art may realize that the following description is illustrative only and is not in any way limiting. Other embodiments of the present invention may readily suggest themselves to such skilled persons having the benefit of this disclosure.

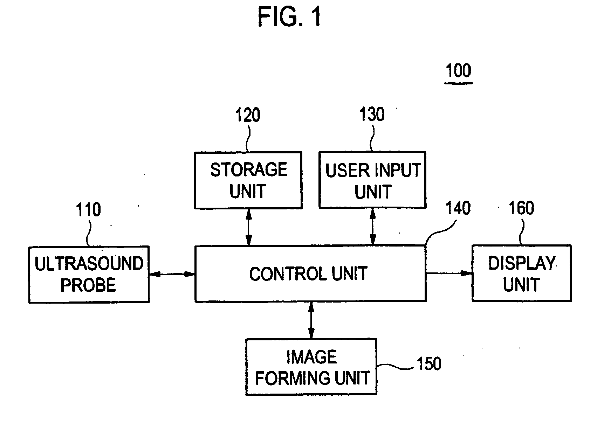

Referring to FIG. 1, an ultrasound system 100 in accordance with an illustrative embodiment is shown. As depicted therein, the ultrasound system 100 may include an ultrasound probe 110 configured to transmit/receive ultrasound signals to/from a fetus in a target object, thereby outputting received signals. In one embodiment, the ultrasound probe 110 may include an endocavity probe and a convex probe. However, the ultrasound probe 110 may not be limited thereto.

The ultrasound system 100 may include a storage unit 120 configured to store a mapping table of image depth information and gestation information. The image depth information may represent information upon a range of an ultrasound image of the target object to be formed in a depth direction. The gestation information may include gestational age information and last menstruation information. However, the gestation information may not be limited thereto. In one embodiment, the image depth information and the gestation information may be set depending on the type of the ultrasound probe. For example, the storage unit 120 may store the mapping table as shown in Table 1.

**Table 1**

| Ultrasound probe | Gestation information | Image depth information |
|---|---|---|
| Endocavity probe | 0 ~ 10 weeks | 8cm |
| | 10 ~ 13 weeks | 10cm |
| Convex probe | 0 ~ 12 weeks | 10cm |
| | 12 ~ 20 weeks | 12cm |
| | 20 ~ 30 weeks | 14cm |
| | 30 ~ 40 weeks | 16cm |

The ultrasound system 100 may include a user input unit 130 configured to receive input information from a user. The input information may include first input information for selecting gestation information. The input information may further include second input information for selecting an ultrasound probe among the endocavity probe, the convex probe and the like. However, the input information may not be limited thereto. The input unit 130 may be configured with a control panel, a mouse, a keyboard or the like capable of receiving the input information. However, the input unit 130 may not be limited thereto.

The ultrasound system 100 may include a control unit 140. The control unit 140 may be configured to set an image depth based on the mapping table and the input information.

The ultrasound system 100 may further include an image forming unit 150. The image forming unit 150 may be configured to form the ultrasound image of the fetus by using the received signals under the control of the control unit 140.

The ultrasound system 100 may further include a display unit 160 configured to the ultrasound image formed in the image forming unit 150. The display unit 160 may be one of a liquid crystal display, a cathode ray tube, a plate panel display and the like, which are capable of displaying the ultrasound. However, the display unit 160 may not be limited thereto.

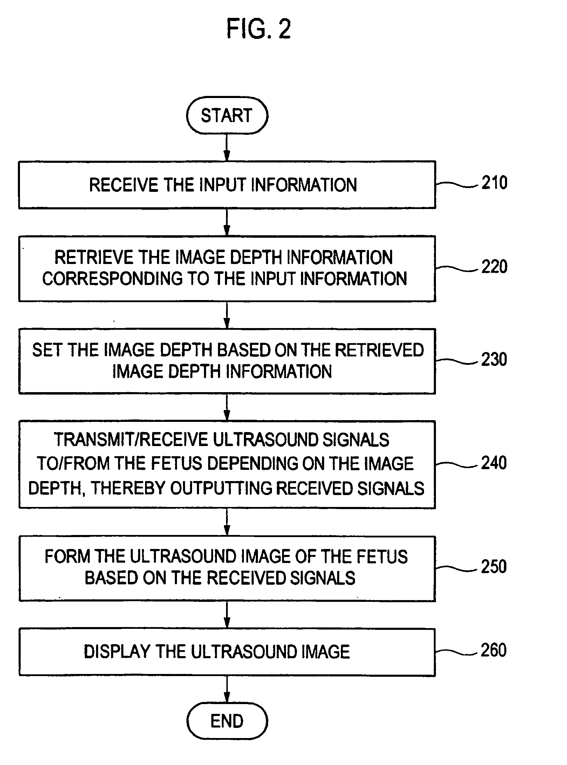

FIG. 2 is a flowchart showing a procedure of setting an image depth. Referring to FIG. 2, the user input unit 130 may receive from a user the input information at block 210.

The control unit 140 may retrieve from the storage unit 120 the image depth information corresponding to the input information at block 220. In one embodiment, the control unit 140 may analyze the input information. If the first input information comprises last menstruation information, the control unit 140 may calculate a gestational age based on the last menstruation information, and retrieve from the storage unit 120 the image depth information corresponding to the calculated gestational age and the input information. Otherwise, if the first input information comprises gestational age information, the control unit 140 may retrieve from the storage unit 120 the image depth information corresponding to the input information.

The control unit 140 may set the image depth based on the retrieved image depth information at block 230. The ultrasound probe 110 may transmit/receive the ultrasound signals to/from the fetus depending on the image depth under the control of the control unit 140, thereby outputting received signals at block 240.

The image forming unit 150 may form the ultrasound image of the fetus by using the received signals under the control of the control unit 140 at block 250. The display unit 160 may display the ultrasound image formed in the image form unit 150 at block

## Claims

1. An ultrasound system (100), comprising:
a storage unit (120) for storing a mapping table of image depth information and gestation information including gestation age information and last menstruation information;
a user input unit (130) configured to receive input information for selecting gestation information from a user; and
a control unit (140) configured to retrieve image depth information corresponding to the input information from the storage unit (120), the control unit (140) being further configured to set an image depth based on the retrieved image depth information.

2. The ultrasound system (100) of Claim 1, wherein the control unit (140) is configured to analyze the input information, if the input information comprises the last menstruation information, then calculate a gestational age based on the last menstruation information and retrieve the image depth information corresponding to the calculated gestational age from the storage unit (120).

3. The ultrasound system (100) of Claim 1, further comprising:
an ultrasound probe (110) configured to transmit/receive ultrasound signals to/from a fetus in a target object to output received signals depending on the image depth; and
an image forming unit (150) configured to form an ultrasound image of the fetus by using the received signals.

4. The ultrasound system (100) of Claim 3, wherein the image depth information and gestation information are set depending on a kind of the ultrasound probe (110).

5. The ultrasound system (100) of Claim 4, wherein the input information includes first input information for selecting gestation information and second input information for selecting the ultrasound probe (110).

6. A method of setting an image in an ultrasound system (100), comprising:
a) receiving input information for selecting gestation information from a user input unit (130) within the ultrasound system (100);
b) retrieving image depth information corresponding to the input information from a storage unit (120) for storing a mapping table of image depth information and gestation information including gestation age information and last menstruation information within the ultrasound system (100); and
c) setting an image depth based on the retrieved image depth information by using a control unit (140) within the ultrasound system (100).

7. The method of Claim 6, wherein the step b) comprises:
analyzing the input information;
if the input information comprises the last menstruation information, then calculating a gestational age based on the last menstruation information; and
retrieving from the storage unit (120) the image depth information corresponding to the calculated gestational age.

8. The method of Claim 6, further comprising:
transmitting/receiving by using an ultrasound probe (110) within the ultrasound system (100) ultrasound signals to/from a fetus in a target object to output received signals depending on the image depth; and
forming by using an image forming unit (150) within the ultrasound system (100) an ultrasound image of the fetus by using the received signals.

9. The method of Claim 6, wherein the image depth information and gestation information are set depending on a type of an ultrasound probe (110).

10. The method of Claim 9, wherein the input information includes first input information for selecting gestation information and second input information for selecting the ultrasound probe (110).

## Patentansprüche

1. Ultraschallsystem (100), welches Folgendes aufweist:
eine Speichereinheit (120) zum Speichern einer Mappingtabelle von Bildtiefeninformationen und Schwangerschaftsinformationen einschließlich Schwangerschaftsalterinformationen und Informationen bezüglich der letzten Menstruation;
eine Benutzereingabeeinheit (130), welche dafür vorgesehen ist,
Eingabeinformationen zum Auswählen von Schwangerschaftsinformationen von einem Benutzer zu empfangen; und
eine Steuereinheit (140), welche dafür vorgesehen ist, Bildtiefeninformationen zu ermitteln, welche den Eingabeinformationen von der Speichereinheit (120) entsprechen, wobei die Steuereinheit (140) des Weiteren dafür vorgesehen ist,
eine Bildtiefe basierend auf den ermittelten Bildtiefeninformationen festzulegen.

2. Ultraschallsystem (100) nach Anspruch 1, wobei die Steuereinheit (140) dafür vorgesehen ist, die Eingabeinformationen zu analysieren, wenn die Eingabeinformationen die Informationen bezüglich der letzten Menstruation beinhalten, dann ein Schwangerschaftsalter basierend auf den Informationen bezüglich der letzten Menstruation zu berechnen und die Bildtiefeninformationen zu ermitteln, welche mit dem berechneten Schwangerschaftsalter von der Speichereinheit (120) korrespondieren.

3. Ultraschallsystem (100) nach Anspruch 1, welches des Weiteren Folgendes aufweist:
eine Ultraschallmesssonde (110), welche dafür vorgesehen ist,
Ultraschallsignale zu einem Fötus in einem Zielobjekt zu übertragen und von demselben zu empfangen, um empfangene Signale abhängig von der Bildtiefe auszugeben; und
eine Bilderzeugungseinheit (150), welche dafür vorgesehen ist, ein Ultraschallbild des Fötus unter Verwendung der empfangenen Signale zu erzeugen.

4. Ultraschallsystem (100) nach Anspruch 3, wobei die Bildtiefeninformationen und Schwangerschaftsinformationen abhängig von einer Art der Ultraschallmesssonde (110) festgelegt werden.

5. Ultraschallsystem (100) nach Anspruch 4, wobei die Eingabeinformationen erste Eingabeinformationen zum Auswählen von Schwangerschaftsinformationen und zweite Eingabeinformationen zum Auswählen der Ultraschallmesssonde (110) beinhalten.

6. Verfahren zum Festlegen eines Bilds in einem Ultraschallsystem (100), welches Folgendes aufweist:
a) Empfangen von Eingabeinformationen zum Auswählen von Schwangerschaftsinformationen von einer Benutzereingabeeinheit (130) innerhalb des Ultraschallsystems (100);
b) Ermitteln von Bildtiefeninformationen, welche den Eingabeinformationen entsprechen, von einer Speichereinheit (120) zum Speichern einer Mappingtabelle von Bildtiefeninformationen und Schwangerschaftsinformationen einschließlich Schwangerschaftsalterinformationen und Informationen bezüglich der letzten Menstruation innerhalb des Ultraschallsystems (100); und
c) Festlegen einer Bildtiefe basierend auf den ermittelten Bildtiefeninformationen unter Verwendung einer Steuereinheit (140) innerhalb des Ultraschallsystems (100).

7. Verfahren nach Anspruch 6, wobei der Schritt b) Folgendes aufweist:
Analysieren der Eingabeinformationen;
falls die Eingabeinformationen die Informationen bezüglich der letzten Menstruation beinhalten, dann Berechnen eines Schwangerschaftsalters basierend auf den Informationen bezüglich der letzten Menstruation; und
Ermitteln von der Speichereinheit (120) die Bildtiefeninformationen, welche mit dem berechneten Schwangerschaftsalter korrespondieren.

8. Verfahren nach Anspruch 6, welches des Weiteren Folgendes aufweist:
Übertragen von Ultraschallsignalen zu einem Fötus und Empfangen derselben von einem Fötus in einem Zielobjekt unter Verwendung einer Ultraschallmesssonde (110) innerhalb des Ultraschallsystems (100), um empfangene Signale abhängig von der Bildtiefe auszugeben; und
Erzeugen unter Verwendung einer Bilderzeugungseinheit (150) innerhalb des Ultraschallsystems (100) eines Ultraschallbilds des Fötus unter Verwendung der empfangenen Signale.

9. Verfahren nach Anspruch 6, wobei die Bildtiefeninformationen und Schwangerschaftsinformationen abhängig von einer Art der Ultraschallmesssonde (110) festgelegt werden.

10. Verfahren nach Anspruch 9, wobei die Eingabeinformationen erste Eingabeinformationen zum Auswählen von Schwangerschaftsinformationen und zweite Eingabeinformationen zum Auswählen der Ultraschallmesssonde (110) beinhalten.

## Revendications

1. Système à ultrasons (100) comprenant :
une unité de stockage (120) pour stocker une table topographique d'informations de profondeur d'image et d'informations de gestation comprenant des informations d'âge de gestation et des informations de dernière menstruation;
une unité d'entrée utilisateur (130) configurée pour recevoir des informations d'entrée pour sélectionner des informations de gestation fournies par une utilisatrice, et
une unité de commande (140) configurée pour récupérer des informations de profondeur d'image correspondant à l'information d'entrée issue de l'unité de stockage (120), l'unité de commande (140) étant en outre configurée pour régler une profondeur d'image sur la base des informations de profondeur d'image récupérées.

2. Système à ultrasons (100) de la revendication 1, dans lequel l'unité de commande (140) est configurée pour analyser les informations d'entrée, si les informations d'entrée comprennent les informations de la dernière menstruation, puis pour calculer un âge de gestation sur la base de l'information de la dernière menstruation, et pour récupérer les informations de profondeur d'image correspondant à l'âge de gestation calculé fourni par l'unité de stockage (120).

3. Système à ultrasons (100) de la revendication 1, comprenant en outre :
une sonde ultrasonique (110) configurée pour émettre/recevoir des signaux ultrasoniques vers/d'un foetus dans un objet cible pour sortir des signaux reçus en fonction de la profondeur d'image, et
une unité de formation d'image (150) configurée pour former une image ultrasonique du foetus en utilisant les signaux reçus.

4. Système à ultrasons (100) de la revendication 3, dans lequel les informations de profondeur d'image et les informations de gestation sont réglées en fonction du type de sonde ultrasonique (110).

5. Système à ultrasons (100) de la revendication 4, dans lequel les informations d'entrée incluent une première information d'entrée utilisée pour sélectionner une information de gestation et une seconde information d'entrée utilisée pour sélectionner la sonde ultrasonique (110).

6. Procédé de réglage d'une image dans un système à ultrasons (100), comprenant:
a) la réception d'informations d'entrée pour sélectionner des informations de gestation issues d'une unité d'entrée utilisateur (130) incluse dans le système à ultrasons (100);
b) la récupération d'informations de profondeur d'image correspondant à l'information d'entrée issue d'une unité de stockage (120) pour stocker une table topographique d'informations de profondeur d'image et d'informations de gestation comprenant des informations d'âge de gestation et des informations de dernière menstruation incluse dans le système à ultrasons (100); et
c) le réglage d'une profondeur d'image sur la base de l'information de profondeur d'image récupérée en utilisant une unité de commande (140) incluse dans le système à ultrasons (100).

7. Procédé de la revendication 6, dans lequel l'étape b) comprend:
l'analyse de l'information d'entrée ;
puis, si l'information d'entrée comprend l'information de dernière menstruation, le calcul d'un âge de gestation sur la base de l'information de dernière menstruation; et
la récupération dans l'unité de stockage (120) de l'information de profondeur d'image correspondant à l'âge de gestation calculé.

8. Procédé de la revendication 6, comprenant en outre :
l'émission/réception, au moyen d'une sonde ultrasonique (110) du système à ultrasons (100), de signaux ultrasoniques vers/d'un foetus dans un objet cible pour sortir des signaux reçus en fonction de la profondeur d'image; et
la formation au moyen d'une unité de formation d'image (150) incluse dans le système à ultrasons (100) d'une image ultrasonique du foetus en utilisant les signaux reçus.

9. Procédé de la revendication 6, dans lequel les informations de profondeur d'image et les informations de gestation sont réglées en fonction du type de sonde ultrasonique (110).

10. Procédé de la revendication 9, dans lequel les informations d'entrée incluent une première information d'entrée utilisée pour sélectionner une information de gestation et une seconde information d'entrée utilisée pour sélectionner la sonde ultrasonique (110).
